# EUROPEAN PATENT SPECIFICATION

(11) **EP 3 634 461 B1**
(45) Date of publication and mention of the grant of the patent: **05.07.2023**
(21) Application number: 18729565.4
(22) Date of filing: 04.05.2018
(51) Int. Cl.: A61K 38/08, A61K 38/45, A61K 39/00, A61P 35/00, C07K 7/06

(54) **VACCINE TARGETING A CRYPTIC TERT EPITOPE, FOR TREATING LUNG CANCER IN A HLA-A*0201-POSITIVE NEVER-SMOKER OR LIGHT-FORMER SMOKER PATIENT**
IMPFSTOFF ZUR ABZIELUNG AUF EIN KRYPTISCHES TERT-EPITOP ZUR BEHANDLUNG VON LUNGENKREBS IN EINEM HLA-A*0201-POSITIVEN PATIENTEN, DER NIE ODER WENIGER GERAUCHT HAT
VACCIN CIBLANT UN ÉPITOPE TERT CRYPTIQUE, POUR LE TRAITEMENT DU CANCER DU POUMON CHEZ UN NON FUMEUR POSITIF AU HLA-A*0201 OU CHEZ UN PATIENT ANCIEN FUMEUR LÉGER

(30) Priority: 09.05.2017 EP 17305527
(43) Date of publication of application: 15.04.2020
(73) Proprietor: Vaxon Biotech, 78160 Marly Le Roi (FR)
(72) Inventor: KOSMATOPOULOS, Kostantinos (Kostas), 75005 Paris (FR); MENEZ-JAMET, Jeanne, 92120 Montrouge (FR)
(74) Representative: Santarelli
(86) International application number: PCT/EP2018/061609
(87) International publication number: WO 2018/206462

(56) References cited:
- WO-A2-2006/120038
- ELENI-KYRIAKI VETSIKA ET AL: "Immunological responses in cancer patients after vaccination with the therapeutic telomerase-specific vaccine Vx-001", CANCER IMMUNOLOGY, IMMUNOTHERAPY, SPRINGER, BERLIN, DE, vol. 61, no. 2, 20 August 2011 (2011-08-20), pages 157-168, XP035005847, ISSN: 1432-0851, DOI: 10.1007/S00262-011-1093-4
- LAN QING ET AL: "Longer Telomere Length in Peripheral White Blood Cells Is Associated with Risk of Lung Cancer and the rs2736100 (CLPTM1L-TERT) Polymorphism in a Prospective Cohort Study among Women in China", PLOS ONE, vol. 8, no. 3, March 2013 (2013-03), XP055415765, ISSN: 1932-6203
- N. A. RIZVI ET AL: "Mutational landscape determines sensitivity to PD-1 blockade in non-small cell lung cancer", SCIENCE, vol. 348, no. 6230, 3 April 2015 (2015-04-03), pages 124-128, XP055322846, ISSN: 0036-8075, DOI: 10.1126/science.aaa1348
- ZHANG TENGFEI ET AL: "The efficacy and safety of anti-PD-1/PD-L1 antibodies for treatment of advanced or refractory cancers: a meta-analysis", ONCOTARGET, vol. 7, no. 45, 8 November 2016 (2016-11-08), pages 73068-73079, XP055415778,
- DOGAN SNJEZANA ET AL: "Molecular Epidemiology of EGFR and KRAS Mutations in 3,026 Lung Adenocarcinomas: Higher Susceptibility of Women to Smoking-Related KRAS-Mutant Cancers", CLINICAL CANCER RESEARCH, vol. 18, no. 22, November 2012 (2012-11), pages 6169-6177, XP055415781,
- BUCCHERI G ET AL: "Karnofsky and ECOG performance status scoring in lung cancer: A prospective, longitudinal study of 536 patients from a single institution", EUROPEAN JOURNAL OF CANCER, ELSEVIER, AMSTERDAM, NL, vol. 32, no. 7, 1 June 1996 (1996-06-01), pages 1135-1141, XP027246652, ISSN: 0959-8049 [retrieved on 1996-06-01]
- TOMOHIRO TAMURA ET AL: "Specific organ metastases and survival in metastatic non-small-cell lung cancer", MOLECULAR AND CLINICAL ONCOLOGY, vol. 3, no. 1, 4 September 2014 (2014-09-04), pages 217-221, XP055497469, GR ISSN: 2049-9450, DOI: 10.3892/mco.2014.410

## Description

### FIELD OF THE INVENTION

The present invention pertains to the field of cancer immunotherapy, and more particularly to the field of antitumor vaccination.

### BACKGROUND AND PRIOR ART

Cancer immunotherapy is intended to stimulate cytotoxic T lymphocytes (CTL) recognizing peptides derived from tumor antigens and presented at the tumor cell surface by HLA class I molecules. CTL-targeted peptides can be dominant or cryptic depending on their affinity for MHC molecules.

Tumor-associated antigens (TAA) are frequently expressed by both tumor cells and normal tissues, contrary to neoantigens that are tumor-specific and most often patient-specific. To circumvent the problem of tolerance to TAA while still targeting an antigen widely expressed by tumors, the inventors proposed a vaccine (Vx-001) targeting a cryptic peptide from TElomerase Reverse Transcriptase (TERT), i.e., a peptide that exhibits a low affinity for the HLA-A*0201 molecule and forms unstable peptide/HLA-I complexes (Menez-Jamet J. *et al.,* 2016). Given the strong correlation between HLA-I affinity and immunogenicity, this cryptic peptide is naturally not immunogenic. To use it as a cancer vaccine, this cryptic peptide was hence optimized to enhance its immunogenicity.

Vx-001 is thus composed of two peptides of nine amino acids: the WT cryptic TERT572 (RLFFYRKSV, SEQ ID No: 1) expressed by tumor cells and its optimized variant TERT572Y (YLFFYRKSV, SEQ ID No: 2). These two peptides are administered separately, along with the adjuvant Montanide ISA51^{®}VG (a mixture of a highly purified mineral oil (Drakeol 6VR) and a surfactant (Mannide monooleate)). The optimized immunogenic TERT572Y is given in the first two vaccinations, in order to trigger a large immune response. The WT TERT572 is given in the following vaccinations, in order to select among all the TERT572Y stimulated T cells, those with the highest specificity for the WT TERT572 that is presented on the surface of tumor cells associated with the HLA-A*0201.

Non small cell lung cancer (NSCLC), including adenocarcinoma, squamous cell carcinoma, and large cell carcinoma, is the most common type of lung cancer, representing about 80% of lung cancer cases; it accounts for approximately 1.2 million new cases annually worldwide.

The clear majority (85%) of cases of lung cancer are due to long-term tobacco smoking. The association of smoking as a risk factor for lung cancer has been established since the 1950s.

Nevertheless, measuring the tobacco exposure for one individual is not obvious since all smokers do not smoke the same amount of cigarettes, and smokers often vary their smoking habits over the years. In addition, there is a degree of individual variability in susceptibility to tobacco mutagens. Several methods were thus proposed to quantify smoking-associated risk factors to stratify cancer patients according to objective parameters.

A first method consists in measuring the amount a person has smoked over a long period of time, using the "pack-year" unit. A pack-year is defined as twenty cigarettes smoked every day for one year. The number of pack-years for one individual is calculated by multiplying the number of packs of cigarettes smoked per day by the number of years the person has smoked this amount of cigarettes each day. For example, 1 pack-year is equal to smoking 20 cigarettes (1 pack) per day for 1 year, or 40 cigarettes per day for half a year, or 10 cigarettes per day during two years, *etc.* Hence, this parameter takes into account not only the duration of smoking, but also its intensity.

Other methods are based on the identification of smoking molecular signatures, which reflect the consequences of smoking in each individual. In a recent study, Rizvi *et al.* demonstrated the existence of a molecular smoking signature based on the amount of transversion mutations in tumor cells (Rizvi NA. *et al.* 2015). This parameter focuses on the effects of smoking rather than on the amount of tobacco smoked by an individual, and distinguishes transversion-high (TH, smoking signature) from transversion-low (TL, never-smoking signature) tumors.

Another molecular smoking signature, based on the amount and/or nature of mutations in *EGFR* and/or KRAS genes, was described by Dogan *et al.* (Dogan S. *et al.* 2012). In this publication, the authors reported that in lung cancers, *EGFR* mutations were far more frequently present in tumors from never-smokers than in those from smokers, contrary to mutations in *KRAS,* which occurred more frequently in smokers. They observed that independently of pack-years, increasing smoking-free years raise the likelihood of *EGFR* mutations. They also showed that the most frequent G>T transversion mutation in smokers (*KRAS* G12C) was more frequent in women and that these women were younger than men with the same mutation, suggesting that women are more susceptible than men to tobacco carcinogens.

About 10-15% of lung cancer cases occur in people who have never or lightly smoked. Lung cancer not related to smoking may be due to other risk factors including environment, hormones, menstrual cycle and viral infection.

Importantly, lung cancer not related to smoking has a worse prognostic than lung cancer related to smoking.

Smoking is highly related to high tumor mutations burden, whereas lung cancer not related to smoking is characterized by a low level of tumor mutations (Rizvi NA., *et al.* 2015). That is why lung cancer in never- or light-smokers is not sensitive to immune check-point inhibitors, contrary to smoking-induced lung cancer.

Vx-001 was tested in a randomized phase IIb clinical trial in metastatic or recurrent stage I-III NSCLC patients who experienced disease control after four cycles of platinum-based chemotherapy.

Patients needed to be HLA-A*0201 positive with a tumor expressing TERT. The primary objective of the study was overall survival. The result of this study was globally not statistically significant, but a detailed analysis of the results, taking into account analysis the smoking history of the patients, as well as other characteristics of the patients such as their specific immune response to the vaccine (immunomonitoring data), their age *etc.,* led to a stratification of the patients with identification of categories of patients for whom vaccination by Vx-001 proved beneficial.

### SUMMARY OF THE INVENTION

The invention is defined in the claims and relates to the selection of patients more likely to respond to vaccination by Vx-001, a vaccine composed of two peptides (SEQ ID No:1 and SEQ ID No: 2) administered separately.

The invention pertains to this vaccine and of each of its components, for use in treating cancer in a HLA-A*0201-positive patient having a non small cell lung cancer (NSCLC) expressing TElomerase Reverse Transcriptase (TERT), wherein said patient is either a never-smoker or a light-smoker, i.e., has been a smoker during less than 25 years or less than 30 years. In case the patient is a light-smoker, the patient is preferably a light-former smoker, i.e., a light-smoker who stopped smoking before NSCLC diagnosis.

The invention also pertains to a theranostic method to determine whether a HLA-A*0201-positive never-smoker or light-smoker patient having a NSCLC expressing TERT is more likely to respond to Vx-001, wherein the patient is more likely to be a good responder if the tumor is non-immunogenic.

### LEGENDS TO THE FIGURES

Figure 1: Vaccination protocol
Figure 2: Response to different tumor antigens of patients with (left) or without (right) natural immunity
Figure 3: Clinical outcome (OS and TTF) of all evaluated patients
Figure 4: Clinical outcome of never-smokers and light former smokers (who have been smoking during less than 25 years). 29 placebo and 26 vaccinated; 11.2 vs 16.2 mo; p=0.07; HR=0.59 (0.32 - 1.06)
Figure 5: Clinical outcome of never-smokers and light former smokers (who have been smoking during less than 25 years) without natural immunity. 16 placebo and 17 vaccinated; 7.9 vs 20.7 mo; p=0.0007; HR=0.29 (0.13 - 0.67)
Figure 6: Clinical outcome of never-smokers and light former smokers (who have been smoking during less than 25 years) who entered the study with a stable disease (SD) after a first-line chemotherapy. 14 placebo and 15 vaccinated. 12.4 vs 24.7 m; p=0.004; HR=0.33 (0.14 - 0.78)
Figure 7: Clinical outcome of male never-smokers and light former smokers (who have been smoking during less than 25 years). 18 placebo and 14 vaccinated. 11.1 vs 24.7 m. p=0.01; HR=0.37 (0.16 - 0.81)

### DETAILED DESCRIPTION OF THE PREFERRED EMBODIMENTS

The references to the methods of treatment by therapy in this description are to be interpreted as references to compounds, pharmaceutical compositions and medicaments of the present invention for use in those methods.

The optimized peptide TERT572Y (peptide of SEQ ID No: 2) induces an anti-tumor CTL response in a HLA-A*0201-positive patient having a NSCLC expressing TERT, when said patient is either a never-smoker or a light-smoker.

When performing anticancer immunotherapy with TERT572Y, vaccination with TERT572Y induces a CTL response against the cryptic peptide TERT572 (SEQ ID No: 1).

The CTL response induced by the initial vaccination with the peptide of SEQ ID No: 2 is then maintained (or amplified) by vaccination with the peptide of SEQ ID No:1.

As used herein, the terms "treat", "treatment" and "treating" refer to any delay of the progression, reduction of severity such as amelioration of quality of life thereof and/or an increase in survival that results from the administration of one or more therapies.

The present invention thus pertains to the use of the native peptide TERT572 (peptide of SEQ ID No: 1), for treating lung cancer in a HLA-A*0201-positive patient having a NSCLC expressing TERT, wherein said patient is either a never-smoker or a light-smoker and wherein said patient has already been vaccinated at least once (and preferably twice) with the peptide of SEQ ID No: 2. In the frame of this immunotherapy treatment, the peptide of SEQ ID No: 1 maintains a CTL immune response initiated by vaccination of the patient with a peptide of SEQ ID No: 2.

The present invention also relates to the use of Vx-001, i.e., the combination of peptides of SEQ ID No: 1 and SEQ ID No: 2, in the treatment of lung cancer in a HLA-A*0201-positive patient having a NSCLC expressing TERT, wherein said patient is either a never-smoker or a light-smoker. As already described, the two peptides of Vx-001 are administered separately. A CTL response against the tumor antigen TERT, more precisely against the cryptic TERT572 peptide of SEQ ID No: 1, is first induced by vaccinating the patient with the peptide of SEQ ID No: 2 and then maintained by vaccinating the patient with the peptide of SEQ ID No: 1.

As used herein, a "smoker" designates an individual who smokes (current smoker) or does not smoke anymore but has been smoking earlier in his life (former smoker), whatever the amount of tobacco smoked each day/week/month; the duration of smoking can correspond to one single period in the individual's life or to the addition of several periods if the individual interrupted his/her smoking habits.

A "never-smoker" (also called a "non-smoker") is an individual who has smoked less than 20 cigarettes in his/her lifetime.

In the present text, a "light-smoker" is an individual who has been a smoker during at most 30 years, preferably at most 25 years. This parameter has been defined as such herein for two reasons: (i) the only available information regarding the smoking history of the patients in the cohort of the phase IIb study was the number of years during which they regularly smoked; and (ii) this parameter led to the stratification of patients into two populations (never-smokers + former light-smokers on the one hand and heavy-former smokers and current smokers on the other hand) whose responses to Vx-001-vaccination were significantly different. Even more significant results were obtained when only the duration of smoking was considered, without considering if the patient is still smoking or not. This led to differentiate never-smokers + light-smokers on the one hand, who better respond to the treatment, and heavy smokers on the other hand, for whom the treatment is not efficient.

Of course, this definition of "light-smokers" can be changed to use any other parameter for measuring tobacco exposure. Using results of another cohort, the skilled artisan can adapt the definition of "light-smokers" to such an alternative way of measuring tobacco exposure. Non-limitative examples of measuring tobacco exposure are:
- smoking pack-years: as mentioned above, this parameter takes into account not only the duration of smoking, but also its intensity. Of course, the skilled artisan can determine, on a cohort of NSCLC patients, an appropriate pack-years threshold to differentiate light-smokers from heavy-smokers in the frame of the present invention.
- molecular smoking signature based on the amount of transversion mutations in tumor cells: as mentioned above, Rizvi *et al.* (2015) distinguished transversion-high (TH, smoking signature) from transversion-low (TL, never-smoking signature) tumors. Of course, the threshold to differentiate the populations (amount of transversions observed in the tumor) can be adapted to include some smokers (with an intermediate number of transversion mutations) into a light-smoker category with never-smokers and determine a heavy-smoking signature for patients exhibiting more transversions. This way of discriminating light- from heavy-smokers is advantageous because it is independent from patients' declarations (which can be biased) and from individual susceptibility to tobacco-induced mutations.
- molecular smoking signature based on the amount and/or nature of mutations in *EGFR* and/or KRAS genes: as mentioned above, Dogan *et al.* (2012) described that a molecular smoking signature based on the analysis of certain genes, such as *EGFR* and *KRAS,* can be defined to segregate NSCLC patients who have mutations frequently caused by tobacco from those who have mutations independent from tobacco (never-smoking + light-smoking). Such a molecular signature can of course be refined by the skilled artisan and adapted to discriminate NSCLC patients to identify those most likely to respond to Vx-001 vaccination, according to the present invention.

According to a particular embodiment of the present invention, Vx-001 is administered to a patient who is a never-smoker or a former smoker who has been a smoker during less than 25 years (or at most 25 years).

According to another particular embodiment, the patient is a never-smoker or a former smoker who stopped smoking at least 5, at least 7, at least 10 or at least 15 years before NSCLC diagnosis.

According to a preferred embodiment illustrated in the experimental part below, the patient first receives 2 vaccinations with the peptide of SEQ ID No: 2 and then 4 vaccinations with the peptide of SEQ ID No:1, with a three weeks interval between said vaccinations ("inducing phase"). Of course, the skilled artisan (physician or clinical investigator) can choose a different vaccination protocol. Possible variations include the number of initial vaccinations with SEQ ID No: 2 (one, two or more) for inducing the CTL response, the interval between the vaccinations (for example, from 1 to 4 weeks or more), the number of vaccinations with SEQ ID No: 1 in this inducing phase (from 1 to 10 or more), and the formulation of the vaccine. In particular, adjuvants different from Montanide can be tested and could possibly necessitate an adaptation of the protocol.

According to another embodiment, after the inducing phase mentioned above, the patient receives additional vaccinations with the peptide of SEQ ID No:1, to maintain the CTL response against TERT ("stabilization phase"). These additional vaccinations can for example be administered every 3 months. They can be administered until relapse. Of course, the skilled artisan (physician or clinical investigator) can choose a different protocol for the stabilization phase. Possible variations include those concerning the interval between the vaccinations (for example, vaccinations can be performed every month, every 2 months, or less frequently, for example every 6 months, especially after a long period of remission), as well as the peptide used. Indeed, the clinician can choose to monitor the CTL response regularly and vaccinate the patient with the peptide of SEQ ID No: 2 again if a decrease in the response is observed. In other words, the clinician can adapt the stabilization phase depending on the patient's response and status.

According to another particular embodiment of the invention, the patient has received a first line chemotherapy, for example a platinum-based chemotherapy, such as a cisplatin-based chemotherapy before the first vaccination with the peptide of SEQ ID No: 2. In this case, the patient preferably has a non-progressing disease, more preferably a stable disease before/upon vaccination with the peptide of SEQ ID No: 2.

Other therapeutic schemes are envisioned in the frame of the present invention. For example, Vx-001 can be administered in an HLA-A*0201-positive never- or light-smoker having a NSCLC expressing TERT either before or during chemotherapy. First-line Vx-001 treatment can also advantageously be envisioned for HLA-A*0201-positive never- or light-smokers having a non-immunogenic tumor expressing TERT. In this case, immunogenic status of the tumor will be determined at diagnosis, for example by detecting tumor antigen specific CTLs using enzyme-linked immunospot (ELISpot) IFNg assay or by any other method such as Tumor Infiltrating Lymphoctes (TILs) detection or gene expression profiling.

As illustrated in the experimental part below, the inventors measured CTL responses against a number of tumor antigens before Vx-001 vaccination. By doing so, they showed that patients having a CTL response against TERT572 before the beginning of the immunotherapy with Vx-001 also have CTL responses against other tumor epitopes. Therefore, they hypothesized that some patients have immunogenic tumors, while others (with no significant CTL response against tumor antigens) have non-immunogenic tumors. They also showed that never- and light-former smokers who have a non-immunogenic tumor respond significantly better to Vx-001 than never- and light-former smokers who have an immunogenic tumor. Hence, a preferred embodiment of the present invention is the use of Vx-001 or of its components in the treatment of NSCLC in a HLA-A*0201-positive never-smoker or light-former smoker patient having a non-immunogenic NSCLC expressing TERT.

In the present text, an "immunogenic tumor" designates a tumor that elicits a significant CTL response against tumor antigens. When performing the present invention, the immunogenicity of the tumor can be assessed by measuring, in a blood sample from the patient, the number of CTLs specific for TERT572 (SEQ ID No: 1) and/or the number of CTLs specific for TERT540 (SEQ ID No: 3), and/or the number of CTLs specific for another tumor antigen. For example, the immunogenicity of the tumor can be assessed by measuring the CTL response against Survivin, which is a universal tumor antigen expressed by a large majority of tumors (Andersen and Thor, 2002). In HLA-A*0201-positive patients considered herein, this response can be detected by measuring, in a blood sample from the patient, the number of CTLs specific for the epitope Survivin96 (SEQ ID No: 5).

*A contrario,* the tumor will be considered as "non-immunogenic" if no CTL response specific for TERT572 (SEQ ID No: 1), TERT540 (SEQ ID No: 3) and/or Survivin96 (SEQ ID No: 5) is detected in a blood sample from the patient. The IFNg ELISpot assay described in the experimental part below can be used to detect T cells specific for the TERT572 peptide or for any other tumor epitope. Of course, as described in the experimental part below, in absence of a significant difference between the response to the tumor antigen tested peptide and the response to an irrelevant peptide, it will be considered that there is no response specific for the tested tumor peptide, *i.e.,* that the tumor is non-immunogenic.

Alternatively, the immunogenicity of the tumor can be assessed by measuring the level of tumor-infiltrating lymphocytes (TILs) in a biopsy from the tumor such as with immunoscore (Pagès F. *et al.,* 2009), or determining the profile of genes expression of the tumor using gene profiling methods (Galon J. *at al.,* 2013, Rizvi NA. *et al.,* 2015, Wang, E. *et al.* 2013).

The inventors found that in some patients, a CTL response against the tumor is detected after chemotherapy, probably because tumor cell lysis following chemotherapy leads to the release of a large amount of tumor epitopes able to induce an antitumor immune response in these patients. These tumor specific CTLs are highly detectable just after the end of chemotherapy, and the amount of CTLs then decreases in the following weeks. The inventors' hypothesis is that this CTL response appears in patients having an immunogenic tumor, who are those who are fully responsive to other immunotherapies such as anti-PD(L)1 treatment. Therefore, for patients who received a chemotherapy treatment such as a platinum-based first line chemotherapy, the immunogenicity status of the tumor will preferably be assessed less than 2 weeks after the end of said chemotherapy, for example less than 7 days after the end of the chemotherapy.

According to a particular embodiment of the present invention, the tumor is considered as non-immunogenic if no CTL response specific for TERT572 (SEQ ID No: 1) is detectable in a blood sample from the patient collected less than 2 weeks, preferably less than 7 days after the end of a platinum-based first line chemotherapy.

According to another particular embodiment of the present invention, the tumor is considered as non-immunogenic if no CTL response specific for TERT540 (SEQ ID No: 3) is detectable in a blood sample from the patient collected less than 2 weeks, preferably less than 7 days after the end of a platinum-based first line chemotherapy.

According to another particular embodiment of the present invention, the tumor is considered as non-immunogenic if no CTL response specific for Survivin96 (SEQ ID No: 5) is detectable in a blood sample from the patient collected less than 2 weeks, preferably less than 7 days after the end of a platinum-based first line chemotherapy.

According to another particular embodiment of the present invention, the patient has a metastatic cancer. The results of the clinical trial indeed show that amongst never- and light-smokers, patients with a metastatic NSCLC (stage IV) respond better to the treatment than patients with a recurrent stage I-III NSCLC.

The results of the clinical trial show that amongst never- and light former-smokers, male patients respond better to the treatment. These results were even more significant when never smokers + all light-smokers (including those who are current smokers and have been smoking during less than 25 years) were considered (Table 7).

According to another particular embodiment of the present invention, the patient has a non-squamous (NSQ) NSCLC. Indeed, the results of the clinical trial show that amongst never- and light-smokers, patients with a non-squamous (NSQ) NSCLC respond better to the treatment.

According to another particular embodiment of the present invention, the patient has an ECOG>0, preferably ECOG=1. The results of the clinical trial indeed show that amongst never- and light-smokers, patients with an ECOG = 1 respond better to the treatment than patients with an ECOG = 0.

According to another aspect, the present invention relates to a method for *in vitro* determining whether a HLA-A*0201-positive never-smoker or light-smoker patient having a TERT-positive NSCLC is more likely to be a good responder to an immunotherapy treatment by vaccination with the peptides of SEQ ID Nos: 1 and 2, comprising assessing the immunogenicity of the tumor, wherein if the tumor is non-immunogenic, the patient is more likely to be a good responder to said immunotherapy treatment.

When performing this method, the immunogenicity of the tumor can be assessed by measuring CTL response specific for the peptide of SEQ ID No: 1 and/or by measuring CTL response specific for the peptide of SEQ ID No: 3 and/or by measuring CTL response specific for the peptide of SEQ ID No: 5 in a blood sample from said individual, wherein if no such CTL response is detectable, the tumor is non-immunogenic.

According to a particular embodiment of the above method, CTL response specific for the peptide of SEQ ID No: 1 or for the peptide of SEQ ID No: 3 or for the peptide of SEQ ID No: 5 or for any other relevant tumor-specific peptide is detected by enzyme-linked immunospot (ELISpot) IFNg assay.

According to another particular embodiment of this method, the immunogenicity of the tumor is assessed less than two weeks after the end of a first-line chemotherapy, preferably less than 7 days after the end of a first-line chemotherapy.

Of course, when performing the theranostic method according to the invention, any other method can be used for assessing the immunogenicity of the tumor. In particular, this can be done by measuring the amount of TILs in a biopsy from the tumor or by gene profiling.

The present invention also pertains to a kit of parts for performing the theranostic method described above, comprising (i) reagents and a plate for performing an ELISpot assay, (ii) a peptide selected amongst SEQ ID No: 1, SEQ ID No: 3 and SEQ ID No: 5 and (iii) an irrelevant peptide as negative control.

Another kit according to the invention comprises materials for detecting a molecular smoking signature in tumor cells. For example, such a kit can comprise a DNA probe for detecting specific mutations in certain genes such as EGFR and KRAS.

Another kit of parts according to the invention comprises the reagents for assessing the immunogenicity of the tumor, as well as materials for detecting a molecular smoking signature in tumor cells.

Other characteristics of the invention will also become apparent in the course of the description which follows of the clinical study and biological assays which have been performed in the framework of the invention and which provide it with the required experimental support, without limiting its scope.

### EXPERIMENTAL RESULTS

### Material and Methods

### Study design and participants

The Vx-001-201 study is a randomized, double blind multicenter study done in 70 centers in France, Germany, Spain, Italy, Greece, Poland, Romania and Czech Republic. Key eligibility criteria were a) non small cell lung cancer (NSCLC), b) stage IV or recurrent stage I-III, c) disease control after platinum based first line chemotherapy according to RECIST 1.1 criteria, d) HLA-A*0201 positivity, e) tumors expressing TElomerase Reverse Transcriptase (TERT), f) ECOG 0 or 1 g) absence of brain metastasis.

The study was conducted according to the Declaration of Helsinki and all applicable regulatory and ethical requirements. The study was approved by the Independent Ethics Committee responsible for each study site in accordance with the local legislation. All patients provided written informed consent.

### Endpoints

a) Principal endpoint: overall survival (OS) measured from random ization
b) Secondary endpoints:
   - Time to Treatment Failure (TTF) measured from random ization
   - OS at 12 months
c) Main exploratory objectives
   - Evaluation of vaccine induced specific immune response
   - Correlation between vaccine specific immune response and clinical response

### Procedures and vaccination protocol

Patients who fulfilled all inclusion criteria were randomized within 4 weeks after the end of first line chemotherapy.

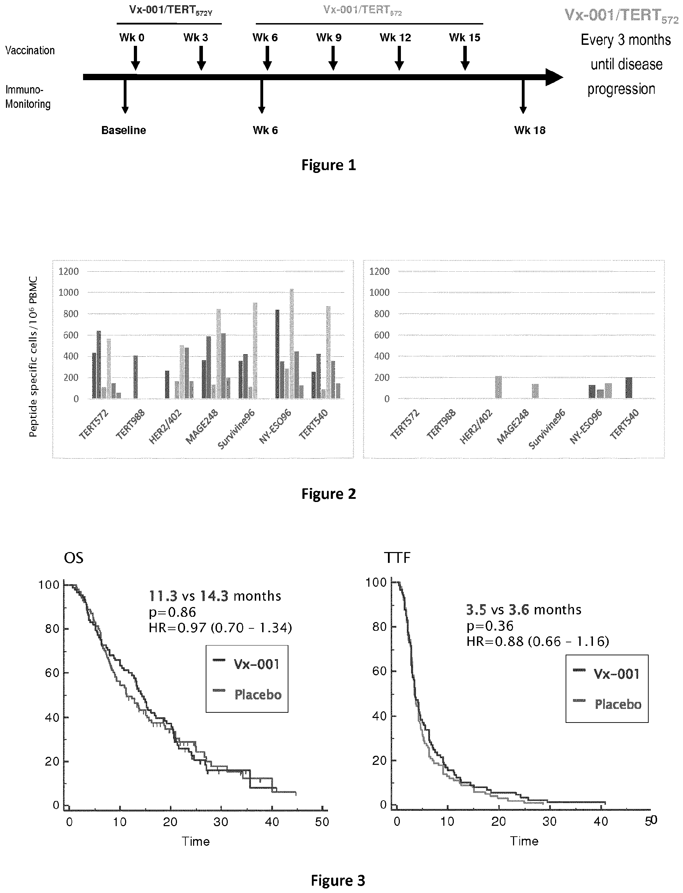

The vaccination protocol consisted in six vaccinations at three weeks interval. The optimized Vx-001/TERT572Y was used in the first two vaccinations and the native Vx-001/TERT572 in the following four vaccinations. Patients who continued controlling their disease after the sixth vaccination received boost vaccination with the Vx-001/TERT572 every three months. Vaccination was stopped at disease progression (Figure 1).

Immune response was evaluated before the first vaccination (baseline), before the third vaccination (W6) and after the sixth vaccination (W18). Patients who received boost vaccinations were monitored for immune response every six months.

### Statistical analysis

To calculate the sample size we estimated the median OS of the placebo arm to be 9.8 months and we expected the median OS in the Vx-001 arm to be 15.2 months. Thus, we needed to randomize 220 patients (including 10% of drop out at the final analysis) to achieve a power of 83% and a one-side alpha of 0.05.

The placebo: Vx-001 ratio was 1 : 1.

The primary and secondary endpoints were analysed in the Full Analysis Set composed of all patients who fulfilled the three five main criteria a) NSCLC, b) stage IV or recurrent stage I-III, and c) disease control after first line chemotherapy, d) HLA-A*0201 positivity and e) TERT expressing tumors.

We used the Kaplan-Meier method to estimate OS and TTF in each arm and a Cox proportional hazards regression model to estimate Hazard Ratio (HR) relating OS and TTF to the treatment effect.

### Immunomonitoring

Immune response was measured using IFNg ELISpot assay to detect T cells specific for the TERT572 peptide. Peripheral Blood Mononuclear Cells (PBMC) were isolated from blood samples collected before vaccination, at W6 (before the third vaccination), W18 (three weeks after the 6^{th} vaccination) and every six months thereafter for patients who received boost vaccinations. PBMC were stored at -160°C and tested when study was unblinded. 2×10⁵ PBMC/well were overnight stimulated with TERT572 or an irrelevant peptide as negative control or the CEF peptides pool as positive control or phytohemmaglutinin (PHA) as aspecific positive control in plates coated with anti IFNg antibody (Diaclone) in AIMV serum free medium (in 6-plicate). The presence of PBMC was evaluated by measuring the number of spots in presence of PHA. The quality of the PBMC was then evaluated by measuring the response to CEF peptides pool (a mixture of polyallelic epitopic peptides from common viruses, flu, HPV and CMV). Samples were considered when they responded to CEF or when all samples from the same patient were unresponsive to CEF (indicating an absence of CEF reactivity due to patient medical history). Number of spots was quantified using a counter and for each conditions the average of the 6 values was calculated. A blood sample was considered responsive to TERT572 or CEF when a) there was a difference higher than 10 spots between the negative control average value and the TERT572 or CEF group average value and b) there was a statistically significant difference between negative control average value and TERT572 or CEF group average value (p<0.05). A patient without pre-vaccination TERT572 response was considered immune responder when a response to TERT572 was detected during the study protocol. Patients with pre-vaccination TERT572 reactivity were considered as immune responders only if (i) this TERT572 reactivity was amplified (at least two folds) after vaccination or if (ii) the patient first lost the pre-vaccination TERT572 reactivity and a new TERT572 reactivity was detected later in the vaccination protocol.

Immune responses to other TERT peptides and to other antigens were also measured using IFNg ELISpot assay. The peptides used to detect T cells are described in Table 1 below.

**Table 1: peptides used to detect specific CTLs**

| Antigen | Peptide | Sequence | SEQ ID No |
|---|---|---|---|
| TERT | TERT 540 | ILAKFLHWL | 3 |
| TERT | TERT 988 | DLQVNSLQTV | 4 |
| Survivin | Survivin-3A 96 | LTLGEFLKL | 5 |
| NY-ESO-1 | NY-ESO-1 157 | SLLMWITQV | 6 |
| HER-2/neu | HER-2/neu402 | TLEEITGYL | 7 |
| MAGE-A | MAGE-A248D9 | YLEYRQVPD | 8 |
| MAGE-A | MAGE-A248G9 | YLEYRQVPG | 9 |

### Results

### Patients

1407 patients were screened and 221 patients were randomized. The main reasons for screening failure were a) patients were HLA-A*0201 negative, b) there was no biopsy material suitable for TERT expression evaluation and c) disease progressed after first line chemotherapy.

Thirty-one patients were excluded from the Full Analysis Set (FAS) analysis because they did not fulfil the main inclusion criteria (25 patients entered the study with progressive disease, 2 patients with a tumor other than NSCLC and 4 patients with no metastatic or recurrent disease). Table 2 shows demographics of the 190 patients of the FAS.

**Table 2: Demographics of evaluated patients. NSQ: non squamous. SQ: squamous. OR: objective response. SD: stable disease ECOG: Eastern Cooperative Oncology Group scale of performance status.**

| | Placebo | | Vx-001 | |
|---|---|---|---|---|
| **All patients** | 101 | | 89 | |

| **Sex** | | | | |
|---|---|---|---|---|
| Male | 72 | 71% | 60 | 67% |
| Female | 29 | 29% | 29 | 33% |

| **Age** | | | | |
|---|---|---|---|---|
| >65 years | 54 | 54% | 45 | 50% |
| <65 years | 47 | 46% | 44 | 50% |

| **Histology** | | | | |
|---|---|---|---|---|
| NSQ | 59 | 59% | 55 | 62% |
| SQ | 38 | 37% | 34 | 38% |
| Mixt | 4 | 4% | 0 | 0% |

| **Response to 1st line chemotherapy** | | | | |
|---|---|---|---|---|
| OR | 52 | 52% | 36 | 40% |
| SD | 49 | 48% | 53 | 60% |

| **ECOG** | | | | |
|---|---|---|---|---|
| 0 | 42 | 42% | 33 | 37% |
| 1 | 59 | 58% | 56 | 63% |

| **Stage** | | | | |
|---|---|---|---|---|
| IV | 89 | 89% | 73 | 82% |
| recurrent | 12 | 11% | 16 | 18% |

| **Smoking status** | | | | |
|---|---|---|---|---|
| Never smoker | 15 | 15% | 10 | 11% |
| Former smoker (all) | 52 | 51% | 63 | 71% |
| Former smoker (≤20 years) | 10 | 10% | 12 | 13% |
| Former smoker (≤25 years) | 14 | 14% | 16 | 18% |
| Former smoker (<30 years) | 17 | 17% | 19 | 21% |
| Former smoker (≥30 years) | 35 | 35% | 44 | 50% |
| Current smoker (including 5 light-smokers (≤25 years)) | 34 | 34% | 16 | 18% |

Patients were randomized within 4 weeks after the end of chemotherapy.

### Immune response in FAS patients

TERT572 specific immune response was detected before vaccination (natural immunity) in 45 out of 166 evaluable patients (27.1%). Percentage of patients with natural immunity was 24.1% and 30.4% in placebo treated and Vx-001 treated patients respectively.

Natural immunity was not limited to TERT572 but extended to other tumor antigens. Blood samples of baseline of six patients with natural immunity and three patients without natural immunity were tested against six additional antigens overexpressed in NSCLC. All patients with natural immunity to TERT572 had T cells reactive to other tumor antigens (such as TERT988, TERT540, MAGE248, HER402, Survivin96, NY-ESO96) while patients without natural immunity to TERT572 did not respond to other tumor antigen and in the rare case a response was detected, it was very weak (Figure 2).

These results strongly suggest that patients with natural immunity have immunogenic tumors while patients without natural immunity have non-immunogenic or poorly immunogenic tumors.

In Vx-001 treated patients, TERT572 specific immune response was evaluated in 79 patients at baseline, 73 patients at W6, 42 patients at W18 and 16 patients who received boost vaccinations. Overall, immune response was measured at least at W6, W18 or thereafter in 75 patients. Twenty-two patients mounted a TERT572 specific immune response (29.3%). Surprisingly this response was significantly more frequent in non-squamous (NSQ) than in squamous (SQ) NSCLC (36% vs 13.3%, p=0.037).

Vaccine-induced immune response was more frequent in patients without natural immunity than in patients with natural immunity even if the frequency was not statistically different (15% vs 36.2% p=0.14).

### Clinical response

Analysis of the FAS patients showed that there was no significant difference in OS between placebo treated and Vx-001 treated patients (11.3 vs 14.3 months, p=0.86, HR=0.97, 95% CI 0.70-1.34). There was significant difference neither in TTF (3.5 vs 3.6 months, p=0.36, HR=0.88, 95% CI 0.66-1.16) nor in 12 months survival (49.5% vs 58%, p=0.24) (Figure 3).

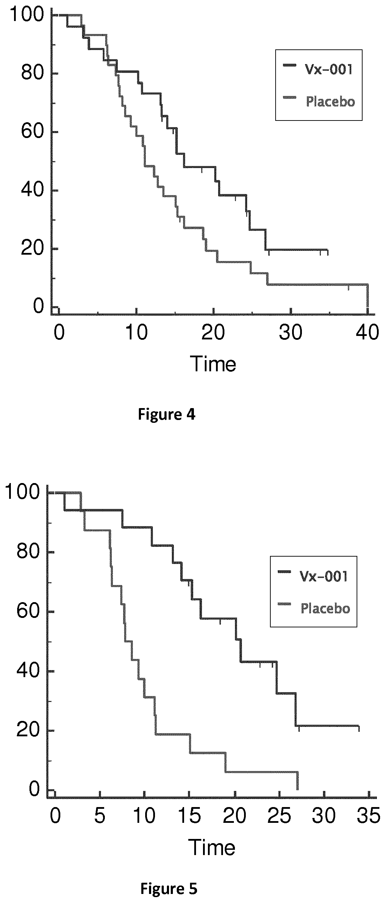

Subgroups analysis showed no significant difference in OS and 12 months survival (table 3) in either subgroup tested excepted the 12 months survival that was significantly higher in Vx-001 treated males (43% vs. 61%, p=0.05) but not in Vx-001 treated females. Moreover, there was a strong trend to significance in patients who were never smokers or light former smokers (≤25 years) (OS 11.2 vs 16.2, p=0.07) (Figure 4).

**Table 3: Sub-group analysis for OS and 12 months survival**

| Sub-group | Median survival (months) | | P value | HR | 95% CI | 12 months OS (%) | | P value |
|---|---|---|---|---|---|---|---|---|
| | Placebo | Vx-001 | | | | Placebo | Vx-001 | |
| **Histology** | | | | | | | | |
| NSQ (n=118) | 11.3 | 13.4 | 0.56 | 0.88 | 0.58-1.34 | 50 | 55 | 0.58 |
| SQ (n=72) | 11.2 | 14.3 | 0.64 | 1.13 | 0.66-1.93 | 46 | 64 | 0.16 |

| **Response to chemotherapy** | | | | | | | | |
|---|---|---|---|---|---|---|---|---|
| OR (n=87) | 11.1 | 15.3 | 0.64 | 0.89 | 0.54-1.45 | 48 | 69 | 0.07 |
| SD (n=103) | 12.4 | 14.3 | 0.98 | 0.99 | 0.64-1.55 | 51 | 52 | 1 |

| **1st line chemotherapy** | | | | | | | | |
|---|---|---|---|---|---|---|---|---|
| CARBO (n=119) | 12.8 | 14.3 | 0.76 | 1.06 | 0.69-1.63 | 50 | 58.2 | 0.46 |
| CDDP (n=69) | 9.9 | 15 | 0.44 | 0.81 | 0.48-1.32 | 46 | 59 | 0.33 |

| **Age** | | | | | | | | |
|---|---|---|---|---|---|---|---|---|
| >65 years (n=98) | 10 | 15 | 0.29 | 0.78 | 0.50-1.24 | 42.6 | 59 | 0.11 |
| <65 years (n=92) | 15.4 | 13.4 | 0.36 | 1.2 | 0.77-2.00 | 55.3 | 57.7 | 0.83 |

| **Sex** | | | | | | | | |
|---|---|---|---|---|---|---|---|---|
| Male (n=131) | 9.9 | 15 | 0.29 | 0.81 | 0.55-1.20 | 43 | 61 | 0.05 |
| Female (n=59) | 15.8 | 13.2 | 0.16 | 1.52 | 0.82-2.79 | 62 | 53.3 | 0.6 |

| **Smoking status** | | | | | | | | |
|---|---|---|---|---|---|---|---|---|
| Never smokers (NS) (n=25) | 11.4 | 14.1 | 0.52 | 0.76 | 0.32-1.79 | 40 | 70 | 0.22 |
| NS +Former smokers (≤20 years)(n=47) | 11.2 | 15.3 | 0.21 | 0.67 | 0.36-1.26 | 48 | 72.7 | 0.13 |
| NS +Former smokers (≤25 years)(n=55) | 11.2 | 16.2 | 0.07 | 0.59 | 0.32-1.05 | 48.2 | 73 | 0.09 |
| NS + Former smokers (<30 years)(n=61) | 11.2 | 16.2 | 0.17 | 0.67 | 0.38-1.19 | 50 | 69 | 0.19 |
| Current smoker (n=50), including light-smoker (≤25 years)(n=5) | 11.1 | 9 | 0.32 | 1.39 | 0.67-2.87 | 50 | 37.5 | 0.54 |

We then focused the analysis on the population of patients who were never or former light- (≤25 years) smokers. This patient population is not likely to have any benefit from the treatment with immune check-point inhibitors (Reck M. *et al.,* 2016, Zhang T. *et al.,* 2016). Table 4 shows the demographics of this population. There was no major imbalance between placebo and Vx-001 treated patients.

**Table 4: Demographics of never smokers and former light-smokers (who smoked ≤25 years)**

| | **Placebo** | | **Vx001** | |
|---|---|---|---|---|
| **All patients** | **29** | | **26** | |

| **Sex** | | | | |
|---|---|---|---|---|
| Male | 18 | 62% | 14 | 54% |
| Female | 11 | 38% | 12 | 46% |

| **Age** | | | | |
|---|---|---|---|---|
| >65 years | 18 | 62% | 13 | 50% |
| <65 years | 11 | 38% | 13 | 50% |

| **Histology** | | | | |
|---|---|---|---|---|
| NSQ | 21 | 72% | 19 | 73% |
| SQ | 6 | 21% | 7 | 27% |
| Mixt | 2 | 7% | 0 | 0% |

| **Response to 1st line chemotherapy** | | | | |
|---|---|---|---|---|
| OR | 15 | 52% | 11 | 42% |
| SD | 14 | 48% | 15 | 68% |

| **1st line chemotherapy** | | | | |
|---|---|---|---|---|
| CARBO | 20 | 69% | 17 | 65% |
| CDDP | 9 | 31% | 9 | 35% |
| CARBO/CDDP | 0 | 0% | 0 | 0% |

| **Natural immunity** | | | | |
|---|---|---|---|---|
| Yes | 8 | 28% | 6 | 23% |
| No | 16 | 55% | 17 | 65% |
| ND | 5 | 17% | 3 | 12% |

| **Stage** | | | | |
|---|---|---|---|---|
| IV | 24 | 83% | 25 | 96% |
| recurrent | 5 | 17% | 1 | 4% |

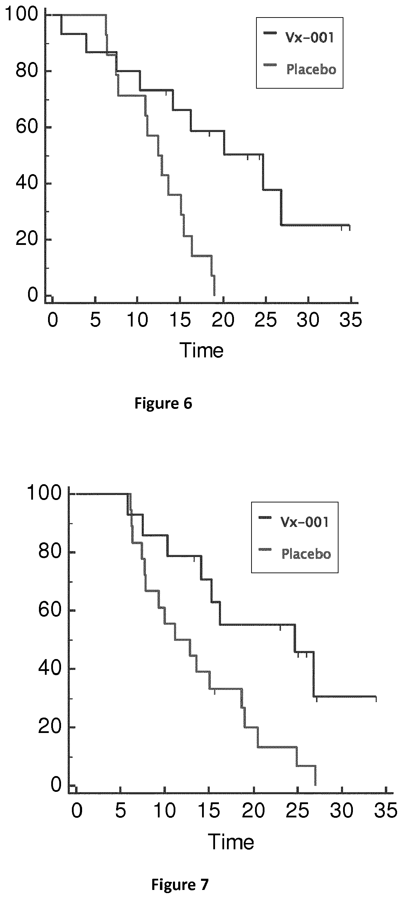

Table 5 shows the clinical outcome of the different sub-groups. Vx-001 significantly prolonged survival in patients without natural immunity (7.9 vs 20.7 mo, p=0.0007, HR=0.29, 95% CI 0.13-0.67) (Figure 5). In this latter population, there was a significant increase of the 12 mo survival (18.7% vs 82.3% p=0.0004). Vx-001 also significantly prolonged survival in patients who entered the study with stable disease (12.4 vs 24.7 mo, p=0.004, HR=0.33, 95% CI 0.14-0.78) (Figure 6) and in males (11.1 vs 24.7 mo, p=0.01, HR=0.37, 95% CI 0.16-0.81) (Figure 7).

**Table 5 Clinical outcome (OS) of sub-groups of never-smoker patients or former light-smokers (former smokers who smoked ≤25 years)**

| **Sub-group** | **Median survival (months)** | | **P value** | **HR** | **95% CI** | **12 months OS %** | | **P value** |
|---|---|---|---|---|---|---|---|---|
| | **Placebo** | **Vx-001** | | | | **Place bo** | **Vx-001** | |
| All (n=55) | 11.2 | 16.2 | 0.07 | 0.59 | 0.32 - 1.06 | 48.3 | 73.1 | 0.09 |

| **Histology** | | | | | | | | |
|---|---|---|---|---|---|---|---|---|
| MH + NSQ (n=42) | 11.1 | 16.2 | 0.06 | 0.53 | 0.26 - 1.04 | 47.8 | 73.7 | 0.12 |
| SQ (n=13) | 11.2 | 14.1 | 0.84 | 0.89 | 0.28 - 2.79 | 50 | 71.4 | 0.59 |

| **Response to chemotherapy** | | | | | | | | |
|---|---|---|---|---|---|---|---|---|
| OR (n=26) | 10 | 15.3 | 0.92 | 0.96 | 0.41 - 2.24 | 40 | 72.7 | 0.13 |
| SD (n=29) | **12.4** | **24.7** | **0.004** | 0.33 | **0.14-0.78** | 57.1 | 73.3 | 0.45 |

| **1st line chemotherapy** | | | | | | | | |
|---|---|---|---|---|---|---|---|---|
| CARBO (n=37) | 10.9 | 15.3 | 0.19 | 0.63 | 0.31 - 1.27 | 40 | 64.7 | 0.19 |
| CDDP (n=18) | 15.1 | 24.7 | 0.18 | 0.50 | 0.17-1.43 | 66.6 | 88.8 | 0.57 |

| **Natural immunity** | | | | | | | | |
|---|---|---|---|---|---|---|---|---|
| Yes (n=14) | 13.6 | 15.3 | 0.86 | 0.90 | 0.27 - 2.93 | 75 | 66.6 | 1 |
| No (n=33) | **7.9** | **20.7** | **0.0007** | **0.29** | **0.13 - 0.67** | **18.7** | **82.3** | **0.0004** |

| **Age** | | | | | | | | |
|---|---|---|---|---|---|---|---|---|
| >65 years (n=31) | 11.2 | 15.3 | 0.27 | 0.64 | 0.29 - 1.39 | 50 | 69.2 | 0.46 |
| <65 years (n=24) | 10.9 | 20.2 | 0.07 | 0.46 | 0.17-1.19 | 45 | 77 | 0.20 |

| **Sex** | | | | | | | | |
|---|---|---|---|---|---|---|---|---|
| Male (n=32) | **11.1** | **24.7** | **0.01** | **0.37** | **0.16 - 0.81** | 50 | 78.6 | 0.14 |
| Female (n=23) | 11.2 | 13.3 | 0.78 | 0.89 | 0.37 - 2.14 | 45.4 | 66.6 | 0.41 |

Regarding the TTF, a significant difference in favor of the Vx-001 was observed in patients without natural immunity (3.1 vs 5.6 mo, p=0.006, HR=0.43 95% CI 0.20-0.92).

**Table 6: Clinical outcome (TTF) of sub-groups of never-smoker patients or former light-smokers (former smokers who smoked ≤ 25 years)**

| **Sub-group** | **Median TTF (months)** | | **P value** | **HR** | **95% CI** |
|---|---|---|---|---|---|
| | Placebo | Vx-001 | | | |
| **Histology** | | | | | |
| MH + NSQ (n=42) | 3.9 | 4.2 | 0.69 | 0.89 | 0.48 - 1.63 |
| SQ (n=13) | 3.0 | 2.9 | 0.69 | 0.81 | 0.27 - 2.46 |

| **Response to chemotherapy** | | | | | |
|---|---|---|---|---|---|
| OR (n=26) | 3.5 | 2.8 | 0.44 | 1.33 | 0.59 - 2.98 |
| SD (n=29) | 3.5 | 3.6 | 0.10 | 0.58 | 0.27 - 1.24 |

| **1st line chemotherapy** | | | | | |
|---|---|---|---|---|---|
| CARBO (n=37) | 3.5 | 2.9 | 0.75 | 0.90 | 0.47 - 1.72 |
| CDDP (n=18) | 3.5 | 5.6 | 0.76 | 0.87 | 034 - 2.21 |

| **Natural immunity** | | | | | |
|---|---|---|---|---|---|
| Yes (n=14) | 4.8 | 2.4 | 0.22 | 1.86 | 0.58 - 5.93 |
| No (n=33) | **3.1** | **5.6** | **0.006** | **0.43** | **0.20** - **0.92** |

| **Age** | | | | | |
|---|---|---|---|---|---|
| >65 years (n=31) | 3.3 | 4.6 | 0.34 | 0.71 | 0.35 - 1.45 |
| <65 years (n=24) | 3.9 | 2.9 | 0.85 | 1.07 | 0.48 - 2.39 |

| **Sex** | | | | | |
|---|---|---|---|---|---|
| Male (n=32) | 3.3 | 2.9 | 0.56 | 0.82 | 0.41 - 1.65 |
| Female (n=23) | 4.8 | 4.2 | 0.96 | 0.98 | 0.43 - 2.22 |

Table 7 shows the clinical outcome of the different sub-groups of never-smoker patients or light-smokers (who smoke ≤ 25 years and stopped smoking or not). Compared to the population studied in tables 4-6 (never smokers + former light-smokers), the population studied in Table 7 comprises 5 additional patients who are light smokers (smoke < 25 years) but who are still smokers.

These results show that Vx-001 significantly prolonged OS in never/light-smokers. This clinical effect was even stronger in never/light-smokers with NSQ histology, with metastatic disease, with ECOG 1, without natural immunity, in men and in patients who entered the study with stable disease.

**Table 7 Clinical outcome (OS) of sub-groups of never-smoker patients and light-smokers (who smoke ≤25 years)**

| Sub-group | OS (months) | | P value | HR | 95% Cl | 12 months OS | | P value |
|---|---|---|---|---|---|---|---|---|
| | Placeb o | Vx-001 | | | | Placeb o | Vx-001 | |
| All (n=60) | **11.2** | **20.2** | **0.037** | **0.55** | **0.32-0.96** | 48.7% | 70.4% | 0.11 |
| | | | | | | | | |

| Histology | | | | | | | | |
|---|---|---|---|---|---|---|---|---|
| NSQ (n=46) | **10.9** | **20.2** | **0.035** | **0.50** | **0.26-0.94** | 32.4% | 70.0% | 0.14 |
| SQ (n=14) | 12.4 | 14.1 | 0.69 | 0.81 | 0.27-2.42 | 57.1% | 71.4% | 0.99 |
| | | | | | | | | |

| Response to treatment | | | | | | | | |
|---|---|---|---|---|---|---|---|---|
| OS (n=28) | 10 | 15.3 | 0.82 | 0.91 | 0.41-2.03 | 41.1% | 63.6% | 0.44 |
| SD (n=32) | **12.4** | **24.7** | **0.005** | **0.34** | **0.15-0.77** | 56.0% | 75.0% | 0.28 |
| | | | | | | | | |

| Stage | | | | | | | | |
|---|---|---|---|---|---|---|---|---|
| IV (n=51) | **11.2** | **16.2** | **0.006** | **0.44** | **0.23-0.82** | 48.0% | 69.2% | 0.16 |
| Recurrent (n=9) | | | | | | | | |
| | | | | | | | | |

| Age | | | | | | | | |
|---|---|---|---|---|---|---|---|---|
| >65y (n=32) | 11.2 | 15.3 | 0.23 | 0.62 | 0.29-1.34 | 50% | 64.3% | 0.48 |
| <65y (n=28) | 10.9 | 20.2 | 0.088 | 0.50 | 0.22-1.13 | 46.7% | 76.9% | 0.13 |
| | | | | | | | | |

| Sex | | | | | | | | |
|---|---|---|---|---|---|---|---|---|
| Male (n=35) | **11.1** | **24.7** | **0.005** | **0.34** | **0.16-0.71** | 47.6% | 78.6% | 0.09 |
| Female (n=25) | 11.2 | 15.3 | 0.90 | 0.95 | 0.41-2.20 | 50.0% | 61.5% | 0.69 |
| | | | | | | | | |

| Natural immunity | | | | | | | | |
|---|---|---|---|---|---|---|---|---|
| Yes (n=15) | 15.4 | 15.3 | 0.81 | 0.87 | 0.27-2.74 | 77.7% | 66.0% | 0.99 |
| No (n=37) | **9.3** | **20.7** | **0.0001** | **0.27** | **0.12-0.58** | **21.0%** | **83.0%** | **0.0002** |
| | | | | | | | | |

| ECOG | | | | | | | | |
|---|---|---|---|---|---|---|---|---|
| 0 (n=28) | 15.1 | 24.7 | 0.15 | 0.52 | 0.22-1.22 | 68.7% | 66.7% | 0.99 |
| 1 (n=32) | **9.3** | **15.3** | **0.048** | **0.49** | **0.23-1.03** | **29.4%** | **73.3%** | **0.030** |

### REFERENCES

Andersen MH and Thor SP. Survivin--a universal tumor antigen. Histol Histopathol. 2002 Apr;17(2):669-75.
Dogan S, Shen R et al. Molecular epidemiology of EGFR and KRAS mutations in 3,026 lung adenocarcinomas: higher susceptibility of women to smoking-related KRAS-mutant cancers. Clin Cancer Res. 2012 Nov 15;18(22):6169-77.
Menez-Jamet J, Gallou C, Rougeot A and Kosmatopoulos K. Optimized tumor cryptic peptides: the basis for universal neo-antigen-like tumor vaccines. Ann Transl Med. 2016 Jul;4(14):266.
Pagès F, Kirilovsky A, Mlecnik B, Asslaber M, Tosolini M, Bindea G, Lagorce C, Wind P, Marliot F, Bruneval P, Zatloukal K, Trajanoski Z, Berger A, Fridman WH, Galon J. In situ cytotoxic and memory T cells predict outcome in patients with early-stage colorectal cancer. J Clin Oncol. 2009 Dec 10;27(35):5944-51.
Rizvi NA, Hellmann MD, Snyder A, Kvistborg P, Makarov V, Havel JJ, Lee W, Yuan J, Wong P, Ho TS, Miller ML, Rekhtman N, Moreira AL, Ibrahim F, Bruggeman C, Gasmi B, Zappasodi R, Maeda Y, Sander C, Garon EB, Merghoub T, Wolchok JD, Schumacher TN, Chan TA. Cancer immunology. Mutational landscape determines sensitivity to PD-1 blockade in non-small cell lung cancer. Science. 2015 Apr 3;348(6230):124-8.
Reck M, Rodríguez-Abreu D, Robinson AG, Hui R, Csőszi T, Fülöp A, Gottfried M, Peled N, Tafreshi A, Cuffe S, O'Brien M, Rao S, Hotta K, Leiby MA, Lubiniecki GM, Shentu Y, Rangwala R, Brahmer JR. et al., *Pembrolizumab versus chemotherapy for PDL-1 positive Non-Small-Cell Lung Cancer.,* N. Engl. J. Med., **2016,** 375, 1823-1833
Wang E, Bedognetti D, Marincola FM. Prediction of response to anticancer immunotherapy using gene signatures. J Clin Oncol. 2013 Jul 1;31(19):2369-71.
Zhang T, Xie J, Arai S, Wang L, Shi X, Shi N, Ma F, Chen S, Huang L, Yang L, Ma W, Zhang B, Han W, Xia J, Chen H, Zhang Y. Zhang et al., The efficacy and safety of anti-PD-1/PD-L1 antibodies for treatment of advanced or refractorycancers: a meta-analysis. Oncotarget, 2016, 7, 73068-73079.

## Claims

1. A peptide of SEQ ID No: 1, for use in the treatment of cancer in a HLA-A*0201-positive patient having a tumor expressing TERT, wherein said patient is either a never-smoker or a light-smoker and wherein said patient has already been vaccinated with the peptide of SEQ ID No: 2.

2. A combination of peptides of SEQ ID No: 1 and SEQ ID No: 2, for use in the treatment of cancer in a HLA-A*0201-positive patient having a tumor expressing TERT, wherein said patient is either a never-smoker or a light-smoker.

3. The peptide or combination of peptides of claim 1 or claim 2, for the use of claim 1 or claim 2, wherein said patient is a never-smoker or a former smoker who has been a smoker during less than 25 years (former light-smoker).

4. The peptide or combination of peptides of claim 1 or claim 2, for the use of any of claims 1 to 3, wherein said patient is a non-smoker or a former smoker who stopped smoking at least 5 years before NSCLC diagnosis.

5. The peptide or combination of peptides of claim 1 or claim 2, for the use of any of claims 1 to 4, wherein said patient has received a first line chemotherapy and has a stable disease before vaccination with the peptide of SEQ ID No: 2.

6. The peptide or combination of peptides of claim 1 or claim 2, for the use of any of claims 1 to 5, wherein said patient has a non-immunogenic tumor.

7. The peptide or combination of peptides of claim 1 or claim 2, for the use of claim 6, wherein the tumor is considered as non-immunogenic if no CTL response specific for the peptide of SEQ ID No: 1 and/or no CTL response specific for the peptide of SEQ ID No: 3 and/or no CTL response specific for the peptide of SEQ ID No: 5 is detectable in a blood sample from the patient before vaccination.

8. The peptide or combination of peptides of claim 1 or claim 2, for the use of claim 7, wherein the blood sample from the patient has been collected less than 2 weeks after the end of a platinum-based first line chemotherapy.

9. The peptide or combination of peptides of claim 1 or claim 2, for the use of any of claims 1 to 8, wherein said patient has a metastatic cancer.

10. The peptide or combination of peptides of claim 1 or claim 2, for the use of any of claims 1 to 9, wherein said patient has a non-squamous (NSQ) NSCLC.

11. The peptide or combination of peptides of claim 1 or claim 2, for the use of any of claims 1 to 10, wherein said patient has an ECOG>0, preferably ECOG=1.

12. A method for *in vitro* determining whether a HLA-A*0201-positive never-smoker or light-smoker patient having a TERT-positive NSCLC is likely to be a good responder to an immunotherapy treatment by vaccination with the peptides of SEQ ID Nos: 1 and 2, comprising assessing the immunogenicity of the tumor, wherein if the tumor is non-immunogenic, the patient is likely to be a good responder to said immunotherapy treatment

13. The method of claim 12, wherein the immunogenicity of the tumor is assessed by measuring CTL response specific for the peptide of SEQ ID No: 1 and/or by measuring CTL response specific for the peptide of SEQ ID No: 3 and/or by measuring CTL response specific for the peptide of SEQ ID No: 5 in a blood sample from said individual before vaccination, wherein if no such CTL response is detectable, the tumor is non-immunogenic.

14. The method of claim 13, wherein the CTL response specific for the peptide of SEQ ID No: 1 and/or the CTL response specific for the peptide of SEQ ID No: 3 and/or the CTL response specific for the peptide of SEQ ID No: 5 is measured by enzyme-linked immunospot (ELISpot) IFNg assay.

## Patentansprüche

1. Peptid der SEQ ID-Nr. 1 zur Verwendung in der Behandlung von Krebs bei einem HLA-A*0201-positiven Patienten mit einem TERT-exprimierenden Tumor, wobei der Patient entweder ein Nieraucher oder ein leichter Raucher ist und wobei der Patient bereits mit dem Peptid der SEQ ID-Nr. 2 geimpft wurde.

2. Kombination von Peptiden der SEQ ID-Nr. 1 und SEQ ID-Nr. 2 zur Verwendung in der Behandlung von Krebs bei einem HLA-A*0201-positiven Patienten mit einem TERT-exprimierenden Tumor, wobei der Patient entweder ein Nieraucher oder ein leichter Raucher ist.

3. Peptid oder Kombination von Peptiden nach Anspruch 1 oder Anspruch 2 zur Verwendung nach Anspruch 1 oder Anspruch 2, wobei der Patient ein Nieraucher oder ein ehemaliger Raucher ist, der weniger als 25 Jahre lang Raucher war (ehemaliger leichter Raucher).

4. Peptid oder Kombination von Peptiden nach Anspruch 1 oder Anspruch 2 zur Verwendung nach einem der Ansprüche 1 bis 3, wobei der Patient ein Nichtraucher oder ein ehemaliger Raucher ist, der mindestens 5 Jahre vor der NSCLC-Diagnose mit dem Rauchen aufgehört hat.

5. Peptid oder Kombination von Peptiden nach Anspruch 1 oder Anspruch 2 zur Verwendung nach einem der Ansprüche 1 bis 4, wobei der Patient eine Erstlinien-Chemotherapie erhalten hat und vor Impfung mit dem Peptid der SEQ ID-Nr. 2 eine stabile Erkrankung hat.

6. Peptid oder Kombination von Peptiden nach Anspruch 1 oder Anspruch 2 zur Verwendung nach einem der Ansprüche 1 bis 5, wobei der Patient einen nichtimmunogenen Tumor hat.

7. Peptid oder Kombination von Peptiden nach Anspruch 1 oder Anspruch 2 zur Verwendung nach Anspruch 6, wobei der Tumor als nicht-immunogen erachtet wird, wenn in einer Blutprobe des Patienten vor Impfung keine für das Peptid der SEQ ID-Nr. 1 spezifische CTL-Antwort und/oder keine für das Peptid der SEQ ID-Nr. 3 spezifische CTL-Antwort und/oder keine für das Peptid der SEQ ID-Nr. 5 spezifische CTL-Antwort nachweisbar ist.

8. Peptid oder Kombination von Peptiden nach Anspruch 1 oder Anspruch 2 zur Verwendung nach Anspruch 7, wobei die Blutprobe des Patienten weniger als 2 Wochen nach dem Ende einer platinbasierten Erstlinien-Chemotherapie entnommen wurde.

9. Peptid oder Kombination von Peptiden nach Anspruch 1 oder Anspruch 2 zur Verwendung nach einem der Ansprüche 1 bis 8, wobei der Patient einen metastasierenden Krebs hat.

10. Peptid oder Kombination von Peptiden nach Anspruch 1 oder Anspruch 2 zur Verwendung nach einem der Ansprüche 1 bis 9, wobei der Patient einen nichtplattenepithelialen (non-squamous, NSQ) NSCLC hat.

11. Peptid oder Kombination von Peptiden nach Anspruch 1 oder Anspruch 2 zur Verwendung nach einem der Ansprüche 1 bis 10, wobei der Patient einen ECOG>0, vorzugsweise ECOG=1, hat.

12. Verfahren zur *In-vitro*-Bestimmung, ob ein HLA-A*0201-positiver Nieraucher- oder Leichtraucher-Patient mit einem TERT-positiven NSCLC wahrscheinlich gut auf eine Immuntherapiebehandlung durch Impfung mit den Peptiden der SEQ ID-Nr. 1 und 2 ansprechen wird, das das Beurteilen der Immunogenität des Tumors umfasst, wobei der Patient wahrscheinlich gut auf die Immuntherapiebehandlung ansprechen wird, wenn der Tumor nicht-immunogen ist.

13. Verfahren nach Anspruch 12, wobei die Immunogenität des Tumors durch Messen der für das Peptid der SEQ ID-Nr. 1 spezifischen CTL-Antwort und/oder durch Messen der für das Peptid der SEQ ID-Nr. 3 spezifischen CTL-Antwort und/oder durch Messen der für das Peptid der SEQ ID-Nr. 5 spezifischen CTL-Antwort in einer Blutprobe des Individuums vor Impfung beurteilt wird, wobei der Tumor nicht-immunogen ist, wenn keine solche CTL-Antwort nachweisbar ist.

14. Verfahren nach Anspruch 13, wobei die für das Peptid der SEQ ID-Nr. 1 spezifische CTL-Antwort und/oder die für das Peptid der SEQ ID-Nr. 3 spezifische CTL-Antwort und/oder die für das Peptid der SEQ ID-Nr. 5 spezifische CTL-Antwort durch Enzyme-Linked Immunospot- (ELISpot) IFNg-Assay gemessen wird.

## Revendications

1. Peptide de SEQ ID No : 1, pour utilisation dans le traitement d'un cancer chez un patient positif au HLA-A*0201 ayant une tumeur exprimant la TERT, dans lequel ledit patient est soit une personne n'ayant jamais fumé soit un fumeur léger et dans lequel ledit patient a déjà été vacciné avec le peptide de SEQ ID No : 2.

2. Combinaison de peptides de SEQ ID No : 1 et de SEQ ID No : 2, pour utilisation dans le traitement d'un cancer chez un patient positif au HLA-A*0201 ayant une tumeur exprimant la TERT, dans lequel ledit patient est soit une personne n'ayant jamais fumé soit un fumeur léger.

3. Peptide ou combinaison de peptides selon la revendication 1 ou la revendication 2, pour l'utilisation selon la revendication 1 ou la revendication 2, dans lequel ledit patient est une personne n'ayant jamais fumé ou un ancien fumeur qui a été un fumeur pendant moins de 25 ans (ancien fumeur léger).

4. Peptide ou combinaison de peptides selon la revendication 1 ou la revendication 2, pour l'utilisation selon l'une quelconque des revendications 1 à 3, dans lequel ledit patient est un non-fumeur ou un ancien fumeur qui a arrêté de fumer au moins 5 ans avant le diagnostic de NSCLC.

5. Peptide ou combinaison de peptides selon la revendication 1 ou la revendication 2, pour l'utilisation selon l'une quelconque des revendications 1 à 4, dans lequel ledit patient a reçu une chimiothérapie de première ligne et a une maladie stable avant vaccination avec le peptide de SEQ ID No : 2.

6. Peptide ou combinaison de peptides selon la revendication 1 ou la revendication 2, pour l'utilisation selon l'une quelconque des revendications 1 à 5, dans lequel ledit patient a une tumeur non-immunogène.

7. Peptide ou combinaison de peptides selon la revendication 1 ou la revendication 2, pour l'utilisation selon la revendication 6, dans lequel la tumeur est considérée comme non immunogène si aucune réponse CTL spécifique au peptide de SEQ ID No : 1 et/ou aucune réponse CTL spécifique au peptide de SEQ ID No : 3 et/ou aucune réponse CTL spécifique au peptide de SEQ ID No : 5 ne sont détectables dans un échantillon sanguin provenant du patient avant vaccination.

8. Peptide ou combinaison de peptides selon la revendication 1 ou la revendication 2, pour l'utilisation selon la revendication 7, dans lequel l'échantillon sanguin provenant du patient a été collecté moins de 2 semaines après la fin d'une chimiothérapie de première ligne basée sur le platine.

9. Peptide ou combinaison de peptides selon la revendication 1 ou la revendication 2, pour l'utilisation selon l'une quelconque des revendications 1 à 8, dans lequel ledit patient a un cancer métastatique.

10. Peptide ou combinaison de peptides selon la revendication 1 ou la revendication 2, pour l'utilisation selon l'une quelconque des revendications 1 à 9, dans lequel ledit patient a un NSCLC non squameux (NSQ).

11. Peptide ou combinaison de peptides selon la revendication 1 ou la revendication 2, pour l'utilisation selon l'une quelconque des revendications 1 à 10, dans lequel ledit patient a un ECOG>0, de préférence ECOG=1.

12. Procédé pour déterminer *in vitro* si un patient n'ayant jamais fumé ou fumeur léger positif au HLA-A*0201 ayant un NSCLC positif à la TERT sera probablement un bon répondant à un traitement d'immunothérapie par vaccination avec les peptides de SEQ ID No : 1 et 2, comprenant l'évaluation de l'immunogénicité de la tumeur, dans lequel si la tumeur est non immunogène, le patient sera probablement un bon répondant audit traitement d'immunothérapie.

13. Procédé selon la revendication 12, dans lequel l'immunogénicité de la tumeur est évaluée en mesurant la réponse CTL spécifique au peptide de SEQ ID No : 1 et/ou en mesurant la réponse CTL spécifique au peptide de SEQ ID No : 3 et/ou en mesurant la réponse CTL spécifique au peptide de SEQ ID No : 5 dans un échantillon sanguin provenant dudit individu avant vaccination, dans lequel si aucune telle réponse CTL n'est détectable, la tumeur est non immunogène.

14. Procédé selon la revendication 13, dans lequel la réponse CTL spécifique au peptide de SEQ ID No : 1 et/ou la réponse CTL spécifique au peptide de SEQ ID No : 3 et/ou la réponse CTL spécifique au peptide de SEQ ID No : 5 sont mesurées par analyse IFNg à immunospot enzymatique (ELISpot).
